(19) Europäisches Patentamt
European Patent Office
Office européen des brevets

(11) Publication number: **0 082 498**
**B1**

(12) # EUROPEAN PATENT SPECIFICATION

(45) Date of publication of patent specification: **02.11.89**

(51) Int. Cl.⁴: **C 07 D 501/20,** C 07 D 499/50, C 07 D 277/38 // C07D277/46, C07D277/42, C07D277/40

(21) Application number: **82111732.2**

(22) Date of filing: **17.12.82**

(54) **Beta-lactam compounds and process for the preparation thereof.**

(30) Priority: **17.12.81 JP 202495/81**
**18.12.81 JP 203597/81**
**24.03.82 JP 45593/82**

(43) Date of publication of application:
**29.06.83 Bulletin 83/26**

(45) Publication of the grant of the patent:
**02.11.89 Bulletin 89/44**

(84) Designated Contracting States:
**CH DE FR GB LI LU NL SE**

(56) References cited:
**EP-A-0 048 954**
**EP-A-0 049 448**
**GB-A-2 076 801**

(73) Proprietor: **SAGAMI CHEMICAL RESEARCH CENTER**
**4-5, Marunouchi 1-chome**
**Chiyoda-ku Tokyo 100 (JP)**

(72) Inventor: **Tunemoto, Daiei**
**2020-59, Midorigaoka**
**Zama-shi Kanagawa-ken (JP)**
Inventor: **Kobori, Takeo**
**3-27-14, Moridai**
**Atsugi-shi Kanagawa-ken (JP)**
Inventor: **Kondo, Kiyosi**
**5-16-4, Chuo-rinkan**
**Yamato-shi Kanagawa-ken (JP)**
Inventor: **Murakami, Yasuo**
**1-27-4, Minamidai Seya-ku**
**Yokohama-shi Kanagawa-ken (JP)**
Inventor: **Toshioka, Tadashi**
**11-806, Mihama-higashi-esteito, 14, Mihama**
**Urayasu-shi Chiba-ken (JP)**
Inventor: **Sakuma, Osamu**
**306, Hijikata-kopo 1230-2**
**Misawa Hino-shi Tokyo (JP)**
Inventor: **Kojima, Etsuko**
**4749-83, Kurohama**
**Hasuda-shi Saitama-ken (JP)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 082 498 B1

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81 (DE)**

**Description**

This invention relates to novel β-lactam compounds and a process for the preparation of these compounds.

Penicillin and cephalosporin compounds show broad antibacterial activities against gram-positive and gram-negative bacteria. Thus, several types of semi-synthetic penicillin and cephalosporin compounds are sold commercially and used as medicines for various infectious diseases in clinial applications. However, there are only a few medicine that can show antibacterial activities against *Pseudomonas aeruginosa, Proteus vulgaris* or the like which are causative of clinically serious infectious diseases. In addition, some of the above-mentioned commercially available agents are relatively unstable against β-lactamase generated by resistant strains of bacteria. These agents are also inadvantageous in that they show only a low antibacterial activity against agent-resistant bacteria, arousing problems in clinical applications (E. E. Wick, Cephalosporins and Penicillins, Chemistry and Biology, E. H. Flynn, Academic Press, New York. N. Y., 1972, Chapter 11). Accordingly, there has been continuous search for β-lactam compounds that have effective antibacterial properties against such pathogens, and there is a need for novel and effective β-lactam compounds.

An object of this invention is to provide β-lactam compounds useful as antibacterial agents.

Another object of this invention is to provide a novel process for preparing such useful β-lactam compounds.

A further object of this invention is to provide intermediate products which are useful for the synthesis of the aforesaid β-lactam compounds or other useful compounds.

The novel β-lactam compounds provided according to this invention are defined by the following general formula [I]

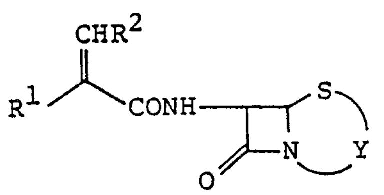

[I]

in which $R^1$ denotes an aminothiazolyl radical or an aminothiazolylmercapto radical; $R^2$ denotes a halogen atom, a cyano radical, a carbamoyl radical, a $C_1$ to $C_4$-alkylmercapto radical or an O-$C_1$ to $C_4$-alkylcarboxyimidoyl radical provided that the case where $R^1$ denotes an aminothiazolyl radical and $R^2$ denotes a halogen atom is excluded; and Y represents a group

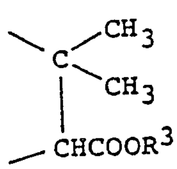

or

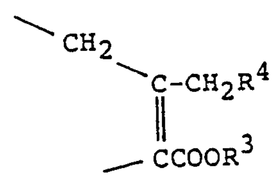

(wherein $R^3$ denotes hydrogen, a salt-forming cation or a protective group for a carboxylic acid, and $R^4$ denotes hydrogen or a residue of a nucleophilic compound).

The inventors have succeeded in synthesizing the β-lactam compounds defined by general formula [I], and found out, as shown in the Test Example mentioned below, that these compounds have a broad antibacterial spectrum and show strong antibacterial activities against not only the gram-positive bacteria but also the gram-negative bacteria, particularly the above-mentioned *Pseudomonas aeruginosa, Proteus vulgaris* and *Escherichia coli*, which arouse problems in clinical applications. It has also been found out that these β-lactam compounds according to this invention show extremely powerful antibacterial activities against the resistive gram-negative bacteria.

The compounds of this invention represented by the above general formula [I] will be explained in more detail as follows.

In the compounds according to this invention, there exist stereoisomers caused by the double bond on the carbon atom to which the substituent radical $R^2$ is bonded.

$R^3$ represents a hydrogen atom, a salt-forming cation or a protective radical for a carboxylic acid. Examples of the cations which $R^3$ represents include those forming alkaline metal salts, alkaline earth metal salts, ammonium salts, etc. The protective radical which $R^3$ represents may be any radicals that are normally used for this purpose in the conventional penicillin and cephalosporin compounds, such as alkyl, lower alkoxymethyl, lower alkylthiomethyl, lower alkanoyloxymethyl, aralkyl, aryl and silyl radicals, etc.

$R^4$ denotes hydrogen or a residue of a nucleophilic compound. The residue of a necleophilic compound may be, for example, hydroxyl; mercapto; optionally substituted lower aliphatic carboxylic acid acyloxy radicals having 2 to 4 carbon atoms such as acetyloxy, propionyloxy, 3-oxobutyryloxy, 3-carboxypropionyloxy, 3-ethyloxycarbamoylpropionyloxy and 4-carboxybutyryloxy; optionally substituted

aromatic carboxylic acid acyloxy radicals such as mandelyloxy, 2-carboxybenzoyloxy, 2-(carboethoxycarmaoyl)benzoyloxy and 2-(carboethoxysulfamoyl)benzoyloxy; carbamoyloxy; phenylglycyloxy, etc. These residues of nucleophilic compounds may further be substituted with alkyl and acyl radicals, etc. In this case, the number of the substituents is normally one or two. Alternatively, $R^4$ the residue of a nucleophilic compound may be that of a quaternary ammonium salt, or may also denote a heterocyclic thio radical, that is, a heterocyclic ring which is bound via S (sulfur atom). In this context, the term "heterocyclic ring" means a 5- or 6-membered ring containing from 1 to 4 hetero atoms selected from the group consisting of O, S and N. The nitrogen atom(s) in the heterocyclic ring may optionally be in the form of N-oxide. Examples of such heterocyclic rings often used include pyridyl, N-oxidepyridyl, pyridazinyl, N-oxidepyridazinyl, pyrazolyl, diazolyl, thiazolyl, thiadiazolyl, oxadiazolyl, triazolyl, tetrazolyl, etc. These heterocyclic rings may optionally have substituents thereon, such as, e.g., lower alkyl having from 1 to 3 carbon atoms, lower alkoxy having from 1 to 3 carbon atoms, halogen atoms, trihalogeno-substituted lower alkyl, hydroxyl, mercapto, amino, carboxyl, carbamoyl, di-lower alkylamino lower alkyl having from 1 to 3 carbon atoms, carboxymethyl, etc. When the heterocyclic ring is substituted by such substituents, the number of substituents is normally one or two. The quaternary ammonium radical represented by $R^4$ may be, for example, pyridinium, methylpyridinium, 3-chloropyridinium, 3-bromopyridinium, 3-iodopyridinium, 4-carbamoylpyridinium, 4-(N-hydroxymethylcarbamoyl)pyridinium, 4-(N-carbomethoxycarbamoyl)pyridinium, 4-(N-cyanocarbamoyl)pyridinium, 4-(carboxymethyl)pyridinium, 4-(hydroxymethyl)pyridinium, 4-(trifluoromethyl)pyridinium, quinolinium, purinium, lutidium, etc.

In view of the antibacterial activity, preferable are β-lactam compounds represented by the following formula [III]:

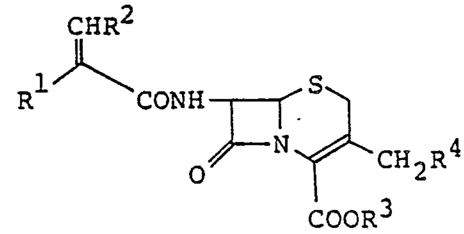

[III]

wherein $R^1$, $R^2$, $R^3$, $R^4$ have the same meanings as in formula [I].

Next, specific compounds according to this invention will be exemplified as follows:

Sodium 7-[2-chloromethylene-3-(2-aminothiazol-5-yl)mercaptoacetamido]-3-(1-methyl-1H tetrazol-5-yl)thiomethyl-3-cephem-4-carboxylate

7-[2-Ethylmercaptomethylene-2-(2-aminothiazol-4-yl)acetamido]-3-(1-methyl-1H tetrazol-5-yl)thiomethyl-3-cephem-4-carboxylic acid (Z- and E-isomer)

7-[2-Methylmercaptomethylene-2-(2-aminothiazol-4-yl)acetamido]-3-(1-methyl-1H tetrazol-5-yl)thiomethyl-3-cephem-4-carboxylic acid (Z- and E-isomer)

7-[2-Cyanomethylene-2-(2-aminothiazol-4-yl)acetamido]-3-(1-methyl-1H tetrazol-5-yl)thiomethyl-3-cephem-4-carboxylic acid (Z-isomer)

7-[2-(O-Methylcarboxyimidoylmethylene)-2-(2-aminothiazol-4-yl)acetamido]-3-(1-methyl-1H tetrazol-5-yl)thiomethyl-3-cephem-4-carboxylic acid

7-[2-Carbamoylmethylene-2-(2-aminothiazol-4-yl)acetamido]-3-(1-methyl-1H tetrazol-5-yl)thiomethyl-3-cephem-4-carboxylic acid

The compounds of this invention can be prepared by reacting a compound having general formula [IV]:

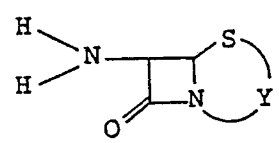

[IV]

(in which Y has the same meaning as defined above), a reactive derivative thereof with respect to the amino radical or a salt thereof, with an acid having general formula [V]:

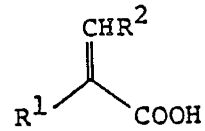

[V]

(in which $R^1$ and $R^2$ have the same meanings as defined above), a reactive derivative thereof with respect to the carboxyl radical or a salt thereof.

The compounds represented by general formula [V], which are used as raw materials for the preparation of the compounds of this invention, can be prepared, for example, by reacting a $R^1$-substituted glyoxylic acid derivative with a Wittig reagent such as halomethylenetriphenylphosphorane; by reacting a

2-formyl acetic acid derivative substituted by $R^1$ radical, with a halogenating agent; or by isomerization reaction of the compound [V].

The acids represented by general formula [IV], which are used as raw materials for the preparation of the compounds according to this invention, are easily available commercially.

Embodiments of the process for preparing the compounds according to this invention will now be described below. When the compounds represented by general formula [V] are to be used as a reactant in the form of free acids (or in the form of salts thereof), it is normally preferable that the reaction can be carried out in the presence of a dehydrating or condensing agent such as N,N'-dicyclohexylcarbodiimide, N-cyclohexyl-N'-morpholinoethylcarbodiimide, N,N'-diethylcarbodiimide, N,N'-carbonyl(2-methylimidazole), trialkyl phosphate, polyphosphoric acid ethyl ester, phosphorus oxychloride, phosphorus trichloride, 2-chloro-1,3,2-dioxaphospholane, oxalyl chloride, etc. Examples of the salts of the compounds represented by general formula [V] include alkaline metal salts, alkaline earth metal salts, ammonium salts, and salts wth organic bases such as triethylamine, dicyclohexylamine, etc.

As the reactive derivatives of the compounds represented by general formula [V], those reactive derivatives which are usually used in the synthesis of acid amines can be used. Examples of such reactive derivatives include acid halides, acid anhydrides, mixed acid anhydrides formed between organic or inorganic acids, active acid amides, acid cyanides, active esters, acid azides, etc. of the compounds represented by general formula [V]. Acid chlorides, mixed acid anhydrides and active amides are particularly preferred.

Examples of the mixed acid anhydrides include those of aliphatic carboxylic acids, aromatic carboxylic acids, alkylcarbonic acids, aralkylcarbonic acids, dialkylphosphoric acids, diphenylphosphoric acids, methanesulfonic acid, p-toluenesulfonic acid and the like. The active esters may be, for example, cyanomethyl esters, substituted phenyl esters, substituted benzyl esters, substituted thienyl esters, etc. The active acid amides may be, for example, imidazole, 4-substituted imidazole, dimethylpyrazole, triazole, tetrazole, saccharin and the like.

The compounds represented by general formula [IV] are subjected to the reaction in the form of free form or in the form of salts or esters thereof. The salts of the compounds represented by general formula [IV] may be, for example, salts with organic acids such as acetates, benzenesulfonates, tartarates, trifluoroacetates, and formates; salts with inorganic acids such as hydrochlorides, hydrobromides, sulfates, and phosphates; alkaline metal salts such as sodium salts and potassium salts; and salts with organic bases such as trimethylamine salts, triethylamine salts and dibutylamine salts. Examples of the esters of the compounds represented by general formula [IV] include benzhydryl esters, trichloroethyl esters, p-nitrobenzyl esters, alkylsilyl esters, etc.

Usually, the above-described reaction for preparing the compounds according to this invention is carried out in the presence of a solvent. The solvents may be any general organic solvents that do not participate in the reaction, such as dimethyl sulfoxide, methyl alcohol, ethyl alcohol, methoxyethanol, diethyl ether, isopropyl ether, benzene, toluene, methylene chloride, chloroform, ethyl acetate, methyl isobutyl ketone, etc. These solvents may be used in admixture with water. In addition, the reaction may be carried out in the presence of a base. Examples of such bases include inorganic bases such as caustic alkalis, alkali carbonates, alkali bicarbonates, and alkali acetates; tertiary amines such as trimethylamine, triethylamine, tributylamine, pyridine, N-methylpiperidine, N-methylmorpholine, lutidine and uridine; secondary amines such as diethylamine, and dicyclohexylamine, etc.

The reaction may be carried out at any temperature. Normally, however, it is conducted with cooling or at room temperature.

Further, the compound represented by formula [I] according to this invention may be prepared by Wittig reaction in which a compound represented by general formula [VI]:

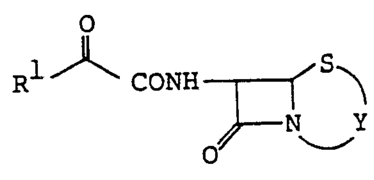

[VI]

(in which $R^1$ and Y have the same meanings as defined above), with a halomethylenetriphenylphosphorane, etc.; by reacting a compound represented by general formula [VII]:

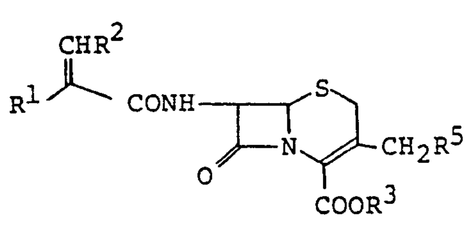

[VII]

(in which $R^1$, $R^2$ and $R^3$ have the same meanings as defined above, and $R^5$ denotes a residue of a nucleophilic compound), with a nucleophilic reagent; or by reacting a compound represented by general formula [VIII]:

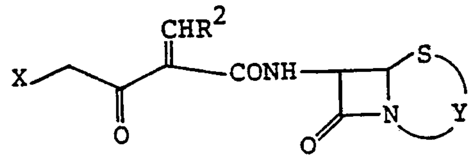

[VIII]

(in which Y has the same meaning as defined above, and X denotes a chlorine atom or a bromine atom), with a thiourea compound.

The β-lactam compounds of this invention prepared in the manner described above may then be purified by a method known to the art, for example, by column chromatography, extraction, precipitation, recrystallization, etc. When necessary, the obtained β-lactam compounds may be converted into a desired salt or an ester by a procedure known to the art.

Intermediate products for use in preparing the compounds of this invention are (2-formyl-2-(2-aminothiazol-4-yl)acetic acid derivatives) having the following general formula [IX]:

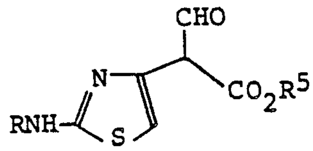

[IX]

in which R has the same meaning as defined above, and $R^5$ denotes an alkyl radical or an aryl radical.

Examples of the compound represented by general formula [IX] include a 2-formyl-2-(2-substituted or unsubstituted aminothiazol-4-yl)acetic acid such as 2-formyl-2-(2-aminothiazol-4-yl)acetic acid, 2-formyl-2-(2-formylaminothiazol-4-yl)acetic acid, 2-formyl-2-(2-acetylaminothiazol-4-yl)acetic acid, 2-formyl-2-(2-chloroacetylaminothiazol-4-yl)acetic acid, 2-formyl-2-(2-trifluoroacetylaminothiazol-4-yl)acetic acid, 2-formyl-2-(2-t-butoxycarbonylaminothiazol-4-yl)acetic acid, 2-formyl-2-(2-tritylaminothiazol-4-yl)acetic acid, 2-formyl-2-(2-trialkylsilylaminothiazol-4-yl)acetic acid; and a 2-formyl-2-(2-substituted or unsubstituted aminothiazol-4-yl)acetate such as methyl, ethyl, t-butyl, benzyl, trichloroethyl, diphenylmethyl, phenyl and trialkylsilyl ester of the above-described compound.

While the chemical structure of the intermediate products has been shown by the structure of general formula [IX] which is a keto-form structure, it may either be represented by general formula [IX'] having an enol-form structure which is in chemical equilibrium with the keto-form structure.

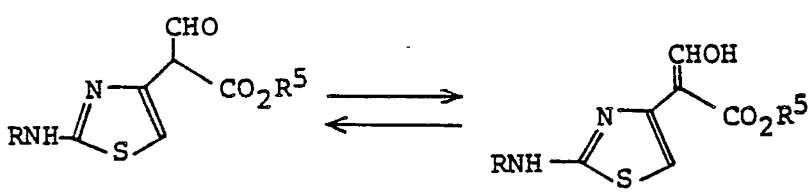

[IX] [IX']

The process for preparing 2-formyl-2-(2-aminothiazol-4-yl)acetic acid derivatives represented by the above general formula [IX] is represented by the following reaction formula:

Method A:

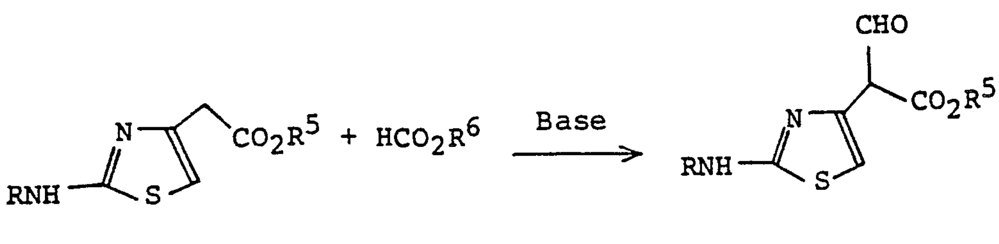


[X] [IX]

Method B:

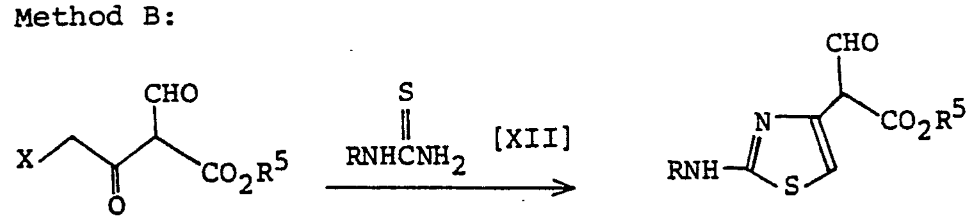

[XII]

[XI] [IX]

Method C:

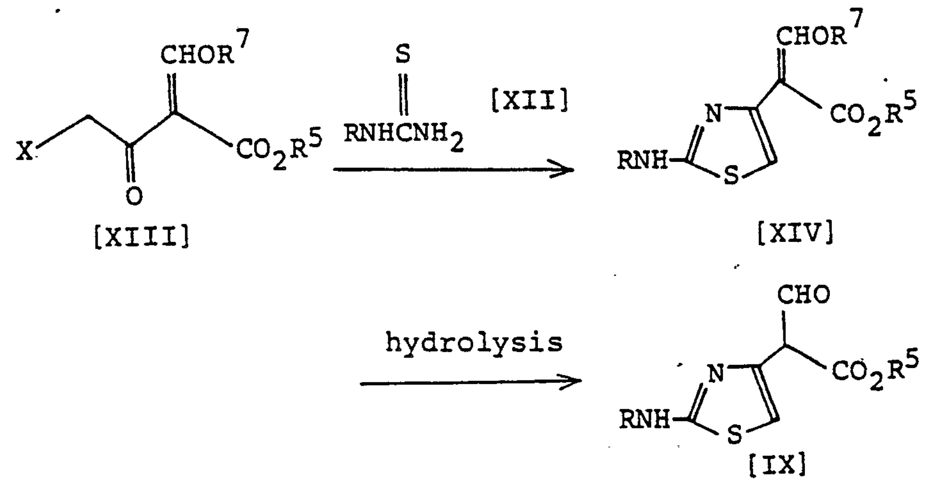


[XII]

[XIII] [XIV]

[IX]

in which $R^5$, R and X have the same meanings as defined above, and $R^6$ and $R^7$ each denote an alkyl radical or an aryl radical.

Embodiments of the process for preparing the intermediate products will now be described below.

[Method A]

This method is to provide the intermediate product represented by general formula [IX] by reacting a 2-(2-aminothiazol-4-yl)acetic acid derivative [X] with a formic acid ester. As to the synthesis of 2-(2-aminothiazol-4-yl)acetic acid derivative represented by general formula [IX], there are described, for example, in "Heterocyclic Compounds: Thiazole and Its Derivatives", Vol. 34, part 2; Edit. by J. V. Metzer, Wiley & Sons, p. 173.

In this method, the presence of a base is indispensable requisites. As the base, there may widely be employed alkali metal such as sodium, potassium; alkali metal hydride such as sodium hydride and potassium hydride; alkali metal alkoxide such as sodium methoxide and potassium t-butoxide; and organic alkali metal compound such as n-butyl lithium and phenyl lithium. Equimolar to several times molar amount of the base with respect to the amount of the compound represented by general formula [X] is sufficient for the amount of the base to be used.

When carrying out the reaction, it is preferable to use a solvent such as hydrocarbons, e.g. hexane, heptane, benzene and toluene; ethers, e.g. diethyl ether, tetrahydrofuran and dimethoxyethane; and polar solvents, e.g. dimethylformamide, dimethyl sulfoxide and hexamethylphosphoric triamide.

The reaction may be conducted at a temperature ranging from 0°C to the reflux temperature of the solvent used.

However, the reaction proceeds smoothly at room temperature without using any special means for cooling or heating.

[Method B]

This method is to provide the intermediate product represented by general formula [IX] by reacting an ester of a γ-halo-α-formylacetoacetic acid represented by general formula [XI] with a thiourea compound represented by general formula [XII]. The compound of formula [XI] can be prepared by a well known method [for example, E. Benary and F. Ebert, Ber., *56B*, 1897 (1923)], and may include, for example, a γ-chloro-α-formylacetoacetic acetate such as methyl γ-chloro-α-formylacetoacetate, ethyl γ-chloro-α-formylacetoacetate, t-butyl γ-chloro-α-formylacetoacetate, benzyl γ-chloro-α-formylacetoacetate, trichloroethyl γ-chloro-α-formylacetoacetate, diphenylmethyl γ-chloro-α-formylacetoacetate, phenyl γ-chloro-α-formylacetoacetate; and γ-bromo-α-formylacetoacetic acetates corresponding to those of the above-mentioned γ-chloro-α-formylacetoacetic acetates.

On the other hand, the thiourea compound represented by general formula [XII] is thiourea or a thiourea derivative, and the thiourea derivative includes, for example, an N-substituted thiourea such as N-(2,2,2-trichloroethoxycarbonyl)thiourea.

When carrying out the reaction, it is preferable to use a solvent such as water; alcoholic solvents, e.g., methanol and ethanol; and ether group solvents, e.g., tetrahydrofuran and dioxane. This reaction may be conducted at room temperature or under heating.

[Method C]

This method is to provide the intermediate product represented by general formula [XIV] by reacting a γ-halo-α-oxymethyleneacetoacetate represented by general formula [XIII], with the thiourea compound represented by formula [XII] (1st Step), and then hydrolyzing the resultant 2-oxymethylene-2-(2-aminothiazol-4-yl)acetic acid derivative represented by general formula [XIV] in an acidic condition (2nd Step). The compound of general formula [XIII] can be prepared by a well known method [for example, E. Benary and F. Ebert, Ber., *56B*, 1897 (1923)], and may include, for example a γ-chloro-α-oxymethylene-acetoacetate such as methyl γ-chloro-α-methoxymethyleneacetoacetate, ethyl γ-chloro-α-methoxy-methyleneacetoacetate, methyl γ-chloro-α-ethoxymethyleneacetoacetate, ethyl γ-chloro-α-ethoxy-methyleneacetoacetate, benzyl γ-chloro-α-methoxymethyleneacetoacetate, benzyl γ-chloro-α-ethoxy-methyleneacetoacetate, trichloroethyl γ-chloro-α-ethoxymethyleneacetoacetate, diphenylmethyl γ-chloro-α-methoxymethyleneacetoacetate, diphenylmethyl γ-chloro-α-ethoxymethyleneacetoacetate; and a γ-bromo-α-oxymethyleneacetoacetates corresponding to those of the above-mentioned γ-chloro-α-oxy-methyleneacetoacetates.

On the other hand, the thiourea compound represented by general formula [XII] is thiourea or a thiourea derivative, and the thiourea derivative includes, for example, an N-substituted thiourea such as N-(2,2,2-trichloroethoxycarbonyl)thiourea.

When carrying out the reaction of the 1st Step, it is preferable to use a solvent. Examples of the solvent includes the solvents exemplified in Method B, and the reaction may be conducted at room temperature or under heating.

The 2nd Step is to hydrolyze 2-oxymethylene-2-(2-aminothiozol-4-yl)acetic acid derivatives under acidic conditions. As the acids to be used, there may be employed, for example, a mineral acid such as hydrochloric acid, sulfuric acid and perchloric acid; an organic acid such as formic acid, acetic acid, methanesulfonic acid and p-toluenesulfonic acid; phosphoric acid; a Lewis acid such as boron trifluoride, aluminum chloride, iron chloride and zinc chloride; a sulfate such as copper sulfate and zinc sulfate; and an acetate such as copper acetate and zinc acetate, etc.

When carrying out the reaction of the 2nd Step, it is preferable to use a solvent. Examples of the solvent includes the solvents exemplified in Method B, and the reaction may be conducted at room temperature or under heating.

As the bacteria and spirochaeta, against which the β-lactam compounds and their non-toxic salts of this invention act effectively, there may be exemplified the following:

*Staphylococcus, Streptococcus, Bacillus* (*Bacillus anthracis,* etc.), *Corynebacterium* (*Corynebacterium diphtheriae,* etc.), *Mycobacterium* (*Mycobacterium tuberculosis,* etc.), *Neisseria* (*Neisseria gonorrhoeae,* etc.), *Pseudomonas* (*Pseudomonas aeruginosa,* etc.), *Escherichia* (*Escherichia coli,* etc.), *Citrobactor, Salmonella, Shigella* (*Shigella dysenteriae,* etc.), *Klebsiella* (*Klebsiella pneumoniae,* etc.), *Enterobactor, Serratia, Proteus, Vibrio* (*Vibrio cholerae,* etc.), *Bordet-Gengou bacillus* (*Haemophilus pertussis,* etc.), *Haemophilus* (*Haemophilus influenzae,* etc.), *Brucella, Haemophilus ducreyi* (*Haemophilus ducreyi*), *Clostridium, Bacteroides, Fusobacterium, Peptococcus, Peptostreptococcus, Eubacterium, Propionibacterium, Spirochete.*

This invention will be explained in more detail by way of the following Examples, Synthesis Examples and Test Example, which however should not be construed to limit this invention.

Synthesis Example 1

(1) 2-Formylamino-5-thiocyanothiazole (925 mg) was suspended in 20 ml of absolute ethanol, and under ice-cooling 204 mg of sodium borohydride was gradually added to the suspension. Then, the temperature of the mixture was returned to room temperature and then the mixture was refluxed further for 10 minutes. After cooling, 6 ml of absolute ethanol containing 337 mg of 85% potassium hydroxide was added to the mixture, and the mixture was stirred at room temperature for 1 to 2 minutes. After dropwise addition of 2.5 ml of absolute ethanol containing 918.5 mg of ethyl bromoacetate, the resulting mixture was stirred at room temperature for 1 hour. Thereafter, water was added to the reaction mixture and pH of the mixture was adjusted to 6.6 with 1N hydrochloric acid, followed by evaporation of the solvent under reduced pressure. The precipitated crystals were collected by filtration, washed and dried to obtain 950 mg of ethyl 3-(2-formylaminothiazol-5-yl)mercaptoacetate as colorless crystals.

Ethyl 3-(2-formylaminothiazol-5-yl)mercaptoacetate:

IR (KBr): 3190, 1730, 1680 $cm^{-1}$.

NMR δppm ($CDCl_3$): 1.27 (t, 3H), 3.48 (s, 1H), 4.19 (q, 1H), 7.52 (s, 1H), 8.61 (s, 1H), 12.2 (bs, 1H).

(2) To 288 mg of 50% sodium hydride was gradually added dropwise 5 ml of ethyl formate at room temperature under stirring. After the dropwise addition, the reaction was completed by heating the mixture at about 45°C. After cooling, 492 mg of 3-(2-formylaminothiazol-5-yl)mercaptoacetic acid was added all at once to the mixture, and the mixture was stirred at room temperature for 1 hour. Then, 10 ml of water and 5 ml of ethyl acetate were added to the reaction mixture and the reaction mixture was separated. The aqueous layer was separated, and the ethyl acetate layer was washed with 2 ml of water. The aqueous layer and washings were combined and adjusted to pH 3.5 with 1N hydrochloric acid. The aqueous layer was extracted with ethyl acetate, and the extract was washed successively with water and a saturated aqueous sodium hydrogen carbonate solution, followed by drying over magnesium sulfate. Evaporation of the solvent yielded 450 mg of ethyl 2-formyl-3-(2-formylaminothiazol-5-yl)mercaptoacetate as pale yellow crystals.

Ethyl 2-formyl-3-(2-formylaminothiazol-5-yl)mercaptoacetate:
IR (KBr): 3190, 1685, 1640 $cm^{-1}$.
NMR δppm ($d_6$-DMSO): 1.22 (t, 3H), 4.11 (q, 2H), 7.45 (s, 1H), 8.08 (s, 1H), 8.46 (s, 1H).

(3) Phosphorus oxychloride (10.96 ml) was added to 2.74 g of ethyl 2-formyl-3-(2-formylaminothiazol-5-yl)mercaptoacetate, and the mixture was stirred at room temperature for 12 to 16 hours. Then, ethyl acetate was added to the mixture, and the mixture was washed with a 5% aqueous sodium hydrogen carbonate solution until the washings became alkaline. Subsequently, the reaction mixture was washed with water and then with a saturated saline solution, and dried over magnesium sulfate. Evaporation of the solvent yielded 2.59 g of ethyl 2-chloromethylene-3-(2-formylaminothiazol-5-yl)mercaptoacetate as pale yellow crystals.

Ethyl 2-chloromethylene-3-(2-formylaminothiazol-5-yl)mercaptoacetate:
IR (KBr): 1700 $cm^{-1}$.
NMR δppm ($d_6$-DMSO): 1.17 (t, 3H), 4.08 (q, 2H), 7.60 (s, 1H), 7.76 (s, 1H), 8.49 (s, 1H).

(4) Ethyl 2-chloromethylene-3-(2-formylaminothiazol-5-yl)mercaptoacetate (1.3 g) was suspended in 10 ml of water, and 20 ml of water containing 880 mg of 85% potassium hydroxide was added dropwise to the suspension at room temperature under stirring. Stirring was continued at room temperature for 15 minutes. Then, the reaction mixture was adjusted to pH 8.8 with 1N hydrochloric acid and washed with ethyl acetate. Then, the ethyl acetate in the aqueous layer was removed by distillation under reduced pressure. The reaction mixture was adjusted to pH 2.0 with 1N hydrochloric acid, and stirred under ice-cooling for 1 hour. The precipitated crystals were collected by filtration, washed with water and dried to obtain 850 mg of 2-chloromethylene-3-(2-formylaminothiazol-5-yl)mercaptoacetic acid as pale yellow crystals.

2-Chloromethylene-3-(2-formylaminothiazol-5-yl)mercaptoacetic acid:
IR (KBr): 1690, 1660 $cm^{-1}$.
NMR δppm ($d_6$-DMSO): 7.59 (s, 1H), 7.72 (s, 1H), 8.49 (s, 1H).

## Example 1

(1) After 842 mg of 2-chloromethylene-3-(2-formylaminothiazol-5-yl)mercaptoacetic acid and 1.43 g of 7-amino-3-(1-methyl-1H-tetrazol-5-yl)thiomethyl-3-cephem-4-carboxylic acid benzhydryl ester were suspended in 30 ml of THF, 715 mg of dicyclohexylcarbodiimido was added to the suspension and the mixture was stirred at room temperature for 2.5 hours. The precipitates were removed by filtration, and the THF was removed by distillation. Then, after ethyl acetate was added to the residue, the precipitates were removed again by filtration. Then, the filtrate was washed successively with a saturated aqueous sodium hydrogen carbonate solution, water and a saturated saline solution, followed by drying over magnesium sulfate. Concentration under reduced pressure followed by purification by column chromatography yielded 1.59 g of 7 - [2 - chloromethylene - 3 - (2 - formylaminothiazol - 5 - yl)mercaptoacetamido] - 3 - (1 - methyl - 1H - tetrazol - 5 - yl)thiomethyl - 3 - cephem - 4 - carboxylic acid benzhydryl ester as yellow amorphous crystals.

7-[2-Chloromethylene-3-(2-formylaminothiazol-5-yl)mercaptoacetamido]-3-(1-methyl-1H-tetrazol-5-yl)thiomethyl-3-cephem-4-carboxylic acid benzhydryl ester:
NMR δppm ($CDCl_3$): 3.72 (bs, 5H), 4.25 (bs, 2H), 4.92 (d, 1H), 5.77 (q, 1H), 6.89 (s, 1H), 7.29 (bs, 10H), 7.57 (s, 1H), 7.60 (s, 1H), 7.85 (d, 1H), 8.42 (s, 1H).

(2) 7 - [2 - Chloromethylene - 3 - (2 - formylaminothiazol - 5 - yl)mercaptoacetamido] - 3 - (1 - methyl - 1H - tetrazol - 5 - yl)thiomethyl - 3 - cephem - 4 - carboxylic acid benzhydryl ester (370 mg) was dissolved in 2.5 ml of anhydrous methylene chloride. Then, 0.75 ml of anisole was added to the solution, and under ice-cooling, 1.5 ml of trifluoroacetic acid was added to the mixture. The resulting mixture was stirred at the same temperature for 2 hours. To the reaction mixture was added 20 ml of ethyl

acetate, and the mixture was washed twice with water and extracted twice with a 5% aqueous sodium hydrogen carbonate solution. The extract was adjusted to pH 1.5 with 1N hydrochloric acid and extracted with ethyl acetate. The resulting extract was washed successively with water and a saturated saline solution, and dried over magnesium sulfate. Evaporation of the solvent yielded 238 mg of 7 - [2 - chloromethylene - 3 - (2 - formylaminothiazol - 5 - yl)mercaptoacetamido] - 3 - (1 - methyl - 1H - tetrazol - 5 - yl)thiomethyl - 3 - cephem - 4 - carboxylic acid as pale yellow crystals.

7-[2-Chloromethylene-3-(2-formylaminothiazol-5-yl)mercaptoacetamido]-3-(1-methyl-1H-tetrazol-5-yl)thiomethyl-3-cephem-4-carboxylic acid:

NMR δppm ($d_6$-DMSO): 3.56 (d, 1H), 3.73 (d, 1H), 3.95 (s, 3H), 4.26 (d, 1H), 4.38 (d, 1H), 5.04 (d, 1H), 5.55 (dd, 1H), 7.22 (s, 1H), 7.63 (s, 1H), 9.44 (s, 1H).

(3) Phosphorus oxychloride (666 mg) was added to 8 ml of absolute methanol under ice-cooling. After the mixture was stirred for 5 minutes, 500 mg of 7 - [2 - chloromethylene - 3 - (2 - formylaminothiazol - 5 - yl)mercaptoacetamido] - 3 - (1 - methyl - 1H - tetrazol - 5 - yl)thiomethyl - 3 - cephem - 4 - carboxylic acid was added thereto, and the resulting mixture was stirred at room temperature further for 1 hour. Then, 8 ml of water was added to the mixture under ice-cooling, and the resulting mixture was adjusted to pH 3.0 with a 10% aqueous sodium hydroxide solution. Subsequently, the reaction mixture was stirred under ice-cooling for 6 hours, and left to stand in a refrigerator for 16 hours. The precipitated crystals were collected by filtration, followed by washing and drying to obtain 330 mg of 7 - [2 - chloromethylene - 3 - (2 - aminothiazol - 5 - yl)mercaptoacetamido] - 3 - (1 - methyl - 1H - tetrazol - 5 - yl)thiomethyl - 3 - cephem - 4 - carboxylic acid as pale yellow crystals.

7-[2-Chloromethylene-3-(2-aminothiazol-5-yl)mercaptoacetamido]-3-(1-methyl-1H-tetrazol-5-yl)thiomethyl-3-cephem-4-carboxylic acid:

IR (KBr): 1770, 1710, 1660, 1620 $cm^{-1}$.

NMR δppm ($d_6$-DMSO): 3.68 (bs, 2H), 3.95 (s, 3H), 4.25 (d, 1H), 4.39 (d, 1H), 5.03 (d, J=5.0Hz, 1H), 5.58 (dd, J=8.0, 5.0Hz, 1H), 7.13 (s, 1H), 7.17 (s, 1H), 7.65 (bs, 2H), 9.39 (d, J=8.0Hz, 1H).

(4) 7 - [2 - Chloromethylene - 3 - (2 - aminothiazol - 5 - yl)mercaptoacetamido] - 3 - (1 - methyl - 1H - tetrazol - 5 - yl)thiomethyl - 3 - cephem - 4 - carboxylic acid (115.9 mg) was dissolved in a solution composed of 2 ml of water and 16.8 mg of sodium hydrogen carbonate, activated charcoal was added to the solution, and the mixture was stirred. After filtration of the mixture through a membrane filter, the filtrate was lyophilized to afford 110 mg of sodium 7 - [2 - chloromethylene - 3 - (2 - aminothiazol - 5 - yl)mercaptoacetamido] - 3 - (1 - methyl - 1H - tetrazol - 5 - yl)thiomethyl - 3 - cephem - 4 - carboxylate as pale yellow crystals (decomposition point: 165 ~ 170°C).

Synthesis Example 2

To a mixture of 44 ml of ethanol and 146 ml of water, there were added 5.5 g of 2-chloromethylene-2-(2-formylaminothiazol-4-yl)acetic acid, 2.2 g of ethyl mercaptan and 2.79 g of sodium hydroxide, and the mixture was stirred at room temperature for 4.5 hours. The reaction mixture was then washed with ethyl acetate, and adjusted to pH 4.5 with 1N hydrochloric acid. The precipitated crystals were collected by filtration, washed with water and dried to obtain 4.4 g of 2-ethylmercaptomethylene-2-(2-formylaminothiazol-4-yl)acetic acid as yellow crystals.

2-Ethylmercaptomethylene-2-(2-formylaminothiazol-4-yl)acetic acid:

NMR δppm ($d_6$-DMSO): 1.28 (t, J=7Hz, 3H), 2.88 (q, J=7Hz, 2H), 7.42 (s, 1H), 7.89 (s, 1H), 8.47 (s, 1H).

Example 2

(1) After 2.52 g of 2-ethylmercaptomethylene-2-(2-formylaminothiazol-4-yl)acetic acid and 4.5 g of 7-amino-3-(1-methyl-1H-tetrazol-5-yl)thiomethyl-3-cephem-4-carboxylic acid benzhydryl ester were dissolved in 160 ml of THF, 2.72 g of dicyclohexylcarbodiimido was added to the solution, and the mixture was stirred at room temperature for 25 hours. After the precipitates were removed by filtration, the filtrate was evaporated to dryness under reduced pressure. The residue was washed with ethyl acetate and dried to obtain 4.35 g of 7 - [2 - ethylmercaptomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid benzhydryl ester as brown amorphous crystals.

7-[2-Ethylmercaptomethylene-2-(2-formylaminothiazol-4-yl)acetamido]-3-(1-methyl-1H-tetrazol-5-yl)thiomethyl-3-cephem-4-carboxylic acid benzhydryl ester:

NMR δppm ($d_6$-DMSO): 1.27 (t, J=7Hz, 3H), 2.89 (q, J=7Hz, 2H), 3.77 (bs, 2H), 3.87 (s, 3H), 4.27 ($q_{AB}$, J=13Hz, 2H), 5.15 (d, J=4Hz, 1H), 5.90 (dd, J=4Hz, 8Hz, 1H), 6.89 (s, 1H), 7.25 (s, 1H), 7.32 (m, 10H), 7.62 (s, 1H), 8.46 (s, 1H), 9.09 (d, J=8Hz, 1H).

(2) 7 - [2 - Ethylmercaptomethylene - 2 - (2 - formylaminothiazol - 4 - yl)acetamido] - 3 - (1 -

methyl - 1H - tetrazol - 5 - yl)thiomethyl - 3 - cephem - 4 - carboxylic acid benzhydryl ester (2.0 g) was dissolved in 10 ml of methylene chloride. Then, 0.88 g of anisole was added to the solution, and 2.6 g of trifluoroacetic acid was added dropwise thereto under ice-cooling, followed by stirring at the same temperature for 3 hours. After evaporation of the solvent from the reaction mixture, benzene was added to the residue. The mixture was adjusted to pH 7.5 with an aqueous sodium hydrogen carbonate solution, and washed with benzene and ethyl acetate, successively. After the mixture was adjusted to pH 5.6 with 1N hydrochloric acid and the precipitates were removed by filtration, the filtrate was adjusted to pH 3.5. Resulting precipitates were removed by filtration, and the filtrate was washed with water and dried to obtain 0.76 g of 7 - [2 - ethylmercaptomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid as yellow amorphous crystals.

7-[2-Ethylmercaptomethylene-2-(2-formylaminothiazol-4-yl)acetamido]-3-(1-methyl-1H-tetrazol-5-yl)thiomethyl-3-cephem-4-carboxylic acid:

NMR δppm ($d_6$-DMSO): 1.29 (t, J=7Hz, 3H), 2.92 (q, J=7Hz, 2H), 3.70 (bs, 2H), 3.95 (s, 3H), 4.33 (bs, 2H), 5.10 (d, J=4Hz, 1H), 5.86 (dd, J=4Hz, 8Hz, 1H), 7.25 (s, 1H), 7.61 (s, 1H), 8.48 (s, 1H), 9.05 (d, J=8Hz, 1H).

(3) To a mixed solution composed of 7 ml of THF, 14 ml of methanol and 0.94 g of phosphorus oxychloride, there was added 0.7 g of 7 - [2 - ethylmercaptomethylene - 2 - (2 - formylaminothiazol - 4 - yl)-acetamido] - 3 - (1 - methyl - 1H - tetrazol - 5 - yl)thiomethyl - 3 - cephem - 4 - carboxylic acid under ice-cooling. Then, the resulting mixture was stirred at the same temperature for 2 hours. After 50 ml of water was added thereto, the mixture was adjusted to pH 3.5 with an aqueous sodium hydrogen carbonate solution, followed by evaporating methanol from the mixture. Then, the residue was adjusted to pH 7.5, washed with ethyl acetate, and adjusted to pH 3.5 with 1N hydrochloric acid. The precipitates were collected by filtration, washed with water and dried to obtain 0.4 g of 7 - [2 - ethylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylic acid as yellow amorphous crystals (decomposition point: 146 ~ 148°C).

7-[2-Ethylmercaptomethylene-2-(2-aminothiazol-4-yl)acetamido]-3-(1-methyl-1H-tetrazol-5-yl)thiomethyl-3-cephem-4-carboxylic acid:

IR (KBr): 1770, 1710, 1620 $cm^{-1}$.

NMR δppm ($d_6$-DMSO): 1.27 (t, J=7Hz, 3H), 2.90 (q, J=7Hz, 2H), 3.70 (bs, 2H), 3.94 (s, 3H), 4.33 ($q_{AB}$, J=13Hz, 2H), 5.10 (d, J=4Hz, 1H), 5.82 (dd, J=4Hz, 8Hz, 1H), 6.60 (s, 1H), 7.08 (bs, 2H), 7.65 (s, 1H), 9.75 (d, J=8Hz, 1H).

(4) 7 - [2 - Ethylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylic acid (100 mg) was dissolved in a solution composed of 2 ml of water and 14 mg of sodium hydrogen carbonate. To the solution was added activated charcoal, and the mixture was stirred and filtrated through membrane filter. Then, the filtrate was lyophilized to obtain sodium 7 - [2 - ethylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylate as yellow amorphous crystals.

Synthesis Example 3

To a mixed solution composed of 24 ml of water, 4.28 g of ethyl mercaptan and 3.87 g of sodium hydroxide, there were added 6.0 g of ethyl 2-chloromethylene-2-(2-formylaminothiazol-4-yl)acetate dissolved in 48 ml of ethanol. After the solution was stirred under refluxing for 2.5 hours, 200 ml of water was added thereto. Then, the mixture was washed with ethyl acetate, followed by adjusting to pH 4.2 with 1N hydrochloric acid. The precipitates were collected by filtration, washed with water and dried to obtain 3.33 g of 2-ethylmercaptomethylene-2-(2-aminothiazol-4-yl)acetic acid (Z- and E-isomer) as yellow amorphous crystals.

2-Ethylmercaptomethylene-2-(2-aminothiazol-4-yl)acetic acid (Z- and E-isomer):

NMR δppm ($d_6$-DMSO): 1.28 (t, J=7Hz, 3H), 2.78 (q, J=7Hz, 2H), 2.85 (q, J=7Hz, 2H), 6.74 (s, 1H), 6.78 (s, 1H), 7.02 (bs, 2H), 7.18 (bs, 2H), 7.82 (s, 1H), 7.90 (s, 1H).

Example 3

(1) After 0.8 g of 2-ethylmercaptomethylene-2-(2-aminothiazol-4-yl)acetic acid (Isomer A and B) and 1.61 g of 7-amino-3-(1-methyl-1H-tetrazol-5-yl)thiomethyl-3-cephem-4-carboxylic acid benzhydryl ester were dissolved in 120 ml of THF, 0.97 g of dicyclohexylcarbodiimido was added to the solution at 25°C. Then, the resulting mixture was stirred at the same temperature for 47 hours. The precipitates were removed by filtration, and the filtrate was concentrated under reduced pressure. The precipitates were collected by filtration, washed successively with ethyl acetate and methylene chloride, and dried to obtain 0.28 g of 7 - [2 - ethylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylic acid benzhydryl ester (Z- and E-isomer) as yellow amorphous crystals.

7-[2-Ethylmercaptomethylene-2-(2-aminothiazol-4-yl)acetamido]-3-(1-methyl-1H-tetrazol-5-yl)thiomethyl-3-cephem-4-carboxylic acid benzhydryl ester (Z- and E-isomer):

NMR δppm ($d_6$-DMSO): 1.26 (t, J=7Hz, 3H), 2.80 (q, J=7Hz, 2H), 3.78 (bs, 2H), 3.88 (s, 3H), 4.30 ($q_{AB}$, J=13Hz, 2H), 5.19 (d, J=4Hz, 1H), 5.96 (dd, J=4Hz, 8Hz, 1H), 6.52 (s, 1H), 6.90 (s, 1H), 7.07 (bs, 1H), 7.38 (m, 10H), 7.56 (s, 1H), 7.68 (s, 1H), 9.64 (d, J=8Hz, 1H).

(2) 7 - [2 - Ethylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylic acid benzhydryl ester (0.23 g, Isomer A and B) was dissolved in 3 ml of methylene chloride. After 0.11 g of anisole was added to the solution, 0.31 g of trifluoroacetic acid was added dropwise thereto under ice-cooling, and the mixture was stirred at the same temperature for further 2.5 hours. After evaporation of the solvent, the residue was adjusted to pH 7.5 with an aqueous sodium hydrogen carbonate solution, washed 3 times with ethyl acetate, and then adjusted to pH 3.6 with 1N hydrochloric acid. The precipitates were collected by filtration, washed with water and dried to obtain 0.05 g of 7 - [2 - ethylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylic acid (Z- and E-isomer) as yellow amorphous crystals.

7-[2-Ethylmercaptomethylene-2-(2-aminothiazol-4-yl)acetamido]-3-(1-methyl-1H-tetrazol-5-yl)thiomethyl-3-cephem-4-carboxylic acid (Z- and E-isomer):

NMR δppm ($d_6$-DMSO): 1.25 (t, 7Hz, 3H), 2.78 (q, J=7Hz, 2H), 3.72 (bs, 2H), 3.94 (s, 3H), 5.12 (d, J=4Hz, 1H), 5.88 (dd, J=4Hz, 8Hz, 1H), 6.51 (s, 1H), 7.07 (bs, 2H), 7.24 (s, 1H), 9.58 (d, J=8Hz, 1H).

## Synthesis Example 4

After 0.75 g of sodium hydroxide was added to 25 g of 15% aqueous methyl mercaptan solution, the resulting mixture was added 4.65 g of ethyl 2-chloromethylene-2-(2-formylaminothiazol-4-yl)acetate dissolved in 37 ml of ethanol. After the reaction mixture was stirred under refluxing for 3 hours, 150 ml of water was added thereto. Then, the mixture was washed with ethyl acetate and adjusted to pH 4.8 with 1N hydrochloric acid. The precipitates were collected by filtration, washed with water and dried to obtain 1.87 g of 2-methylmercaptomethylene-2-(2-aminothiazol-4-yl)acetic acid (Z- and E-isomer) as yellow amorphous crystals.

2-Methylmercaptomethylene-2-(2-aminothiazol-4-yl)acetic acid (Z- and E-isomer):

NMR δppm ($d_6$-DMSO): 2.38 (s, 3H), 2.48 (s, 3H), 6.77 (s, 1H), 6.82 (s, 1H), 7.05 (bs, 2H), 7.19 (bs, 2H), 7.80 (s, 1H), 7.92 (s, 1H).

## Example 4

(1) After 1.5 g of 2-methylmercaptomethylene-2-(2-aminothiazol-4-yl)acetic acid (Isomer A and B) and 3.2 g of 7-amino-3-(1-methyl-1H-tetrazol-5-yl)thiomethyl-3-cephem-4-carboxylic acid benzhydryl ester were dissolved in 240 ml of THF, 1.94 g of dicyclohexylcarbodiimido was added thereto. Then, the mixture was stirred at the temperature of 22 ~ 29°C for 67 hours. After the precipitates were removed by filtration, filtrate was concentrated under reduced pressure. The precipitates were collected by filtration, washed successively with ethyl acetate and methylene chloride, and dried to obtain 1.92 g of 7 - [2 - methylmercaptomethylene - 2 - (2 - aminothiazol - 4 - yl)acetamido] - 3 - (1 - methyl - 1H - tetrazol - 5 - yl)-thiomethyl - 3 - cephem - 4 - carboxylic acid benzhydryl ester (Z- and E-isomer) as brown crystals.

7-[2-Methylmercaptomethylene-2-(2-aminothiazol-4-yl)acetamido]-3-(1-methyl-1H-tetrazol-5-yl)thiomethyl-3-cephem-4-carboxylic acid benzhydryl ester (Z- and E-isomer):

NMR δppm ($d_6$-DMSO): 2.34 (s, 3H), 2.45 (s, 3H), 3.78 (bs, 2H), 3.86 (s, 3H), 4.30 ($q_{AB}$, J=13Hz, 2H), 5.16 (d, J=4Hz, 1H), 5.90 (dd, J=4Hz, 8Hz, 1H), 6.50 (s, 1H), 6.60 (s, 1H), 6.87 (s, 1H), 7.05 (bs, 2H), 7.32 (m, 10H), 7.58 (s, 1H), 9.62 (d, J=8Hz, 1H), 9.78 (d, J=8Hz, 1H).

(2) 7 - [3 - Methylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylic acid benzhydryl ester (1.5 g, Isomer A and B) was dissolved in 10 ml of methylene chloride. After 0.7 g of anisole was added thereto, 2.07 g of trifluoroacetic acid was added dropwise to the mixture under ice-cooling. Then, the mixture was stirred at room temperature for 2.5 hours. After evaporation of the solvent, the residue was adjusted to pH 7.2 with an aqueous sodium hydrogen carbonate solution, washed with ethyl acetate, and adjusted to pH 3.5 with 1N hydrochloric acid. The precipitates were collected by filtration, washed with water and dried to obtain 0.48 g of 7 - [2 - methylmercaptomethylene - 2 - (2 - aminothiazol - 4 - yl)acetamido] - 3 - (1 - methyl - 1H - tetrazol - 5 - yl)thiomethyl - 3 - cephem - 4 - carboxylic acid (Z- and E-isomer) as yellow amorphous crystals.

7-[2-Methylmercaptomethylene-2-(2-aminothiazol-4-yl)acetamido]-3-(1-methyl-1H-tetrazol-5-yl)thiomethyl-3-cephem-4-carboxylic acid (Z- and E-isomer):

NMR δppm ($d_6$-DMSO): 2.35 (s, 3H), 2.47 (s, 3H), 3.70 (bs, 2H), 3.94 (s, 3H), 5.10 (d, J=4Hz, 1H), 5.78 (m, 1H), 6.52 (s, 1H), 6.61 (s, 1H), 7.10 (bs, 2H), 7.23 (bs, 2H), 7.35 (s, 1H), 7.61 (s, 1H).

Synthesis Example 5

(1) After 2.14 g of ethyl (2-formylaminothiazol-4-yl)acetate was dissolved in 50 ml of 1,4-dioxane, 2.22 g of selenium dioxide was added thereto, and the mixture was refluxed for 2 hours. The thermotime reaction mixture was collected by filtration and the filtrate was left to stand over night. The precipitated crystals were collected by filtration, washed with ethyl acetate and dried to obtain 2.12 g of ethyl (2-formylaminothiazol-4-yl)glyoxalate as colorless crystals.

Ethyl (2-formylaminothiazol-4-yl)glyoxalate:
IR (KBr): 1730, 1680 $cm^{-1}$.
NMR δppm ($d_6$-DMSO): 1.35 (t, J=7Hz, 3H), 4.38 (q, J=7Hz, 2H), 8.52 (s, 1H), 8.59 (s, 1H), 12.7 (bs, 1H).

(2) After 1.7 g of ethyl (2-formylaminothiazol-4-yl)glyoxalate was suspended in 35 ml of THF, 2.7 g of cyanomethylenetriphenylphosphorane was added to the suspension. Subsequently, the mixture was stirred at room temperature for 4 hours, and the reaction mixture was concentrated. After the concentrate was passed through the silica gel column chromatography, the resulting eluate was concentrated to obtain ethyl 2-cyanomethylene-2-(2-formylaminothiazol-4-yl)acetate (Z- and E-isomer).

Ethyl 2-cyanomethylene-2-(2-formylaminothiazol-4-yl)acetate:

Isomer A:
NMR δppm ($d_6$-DMSO): 1.33 (t, 3H), 4.32 (q, 2H), 6.59 (s, 1H), 7.84 (s, 1H), 8.56 (s, 1H), 12.4 (bs, 1H).

Isomer B:
NMR δppm ($d_6$-DMSO): 1.38 (t, 3H), 4.43 (q, 2H), 6.65 (s, 1H), 7.76 (s, 1H), 8.56 (s, 1H), 12.4 (bs, 1H).

(3) To the aforementioned ethyl 2-cyanomethylene-2-(2-formylaminothiazol-4-yl)acetate (Z- and E-isomer) was added 28 ml of 3% aqueous potassium hydroxide solution, and the resulting mixture was stirred at room temperature for 1 hour. The insolubles were removed by filtration and the filtrate was washed with ethyl acetate. The residue was adjusted to pH 3.0 with 1N hydrochloric acid and extracted twice with ethyl acetate. The extract was washed twice with water and then washed twice with a saturated saline solution, and dried over magnesium sulfate. The resulting solution was concentrated and the precipitates were collected by filtration to obtain 1.42 g of 2-cyanomethylene-2-(2-formylaminothiazol-4-yl)acetic acid (Z- and E-isomer) as yellow amorphous crystals.

2-Cyanomethylene-2-(2-formylaminothiazol-4-yl)acetic acid (Z- and E-isomer):
IR (KBr): 2210, 1720, 1690, 1640 $cm^{-1}$.

Example 5

(1) After 1.42 g of 2-cyanomethylene-2-(2-formylaminothiazol-4-yl)acetic acid and 2.62 g of 7-amino-3-(1-methyl-1H-tetrazol-5-yl)thiomethyl-3-cephem-4-carboxylic acid benzhydryl ester were suspended in 50 ml of THF, 1.42 g of dicyclohexylcarbodiimido was added to the suspension. Subsequently, the mixture was stirred at room temperature for over night. After the mixture was filtrated, the filtrates were concentrated. The residue was passed through column chromatography to obtain 1.24 g of pale yellow crystals (Z-isomer) and 1.41 g of pale yellow amorphous crystals (E-isomer) of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid benzhydryl ester, respectively.

7-[2-Cyanomethylene-2-(2-formylaminothiazol-4-yl)acetamido]-3-(1-methyl-1H-tetrazol-5-yl)thiomethyl-3-cephem-4-carboxylic acid benzhydryl ester:

Z-isomer:
IR (KBr): 2210, 1770, 1715, 1698, 1680 $cm^{-1}$.
NMR δppm ($d_6$-DMSO): 3.80 (bs, 2H), 3.89 (s, 3H), 4.33 (bs, 2H), 5.25 (d, J=5Hz, 1H), 5.90 (dd, J=8Hz, 5Hz, 1H), 6.16 (s, 1H), 6.94 (s, 1H), 7.40 (m, 10H), 7.73 (s, 1H), 8.56 (s, 1H), 9.72 (d, J=8Hz, 1H), 12.5 (bs, 1H).

E-isomer:
IR (KBr): 2210, 1780, 1715, 1675 $cm^{-1}$.
NMR δppm ($d_6$-DMSO): 3.79 (bs, 2H), 3.87 (s, 3H), 4.31 (bs, 2H), 5.29 (d, J=5Hz, 1H), 5.96 (dd, J=8Hz, 5Hz, 1H), 6.37 (s, 1H), 6.91 (s, 1H), 7.38 (m, 10H), 7.51 (s, 1H), 8.54 (s, 1H), 9.97 (d, J=8Hz, 1H), 12.5 (bs, 1H).

(2) To a mixed solution composed of 1.67 ml of trifluoroacetic acid, 0.84 g of anisole and 2.8 ml of anhydrous methylene chloride, there were added 390 mg of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid benzhydryl ester (E-isomer) under ice-cooling. Then, the resulting mixture was stirred for 50 minutes. After 50 ml of ethyl acetate and 20 ml of 5% aqueous sodium hydroxide solution were added to

the mixture, the mixture was separated. The aqueous layer was separated and extracted twice with 10 ml of ethyl acetate. Then, each ethyl acetate layer was combined, washed successively twice with water and twice with a saturated saline solution, and dried over magnesium sulfate. After the resulting solution was concentrated, the residue was added small amount of benzene and left to stand. The precipitated crystals were collected by filtration, washed with benzene and dried over magnesium sulfate to obtain 280 mg of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid (Isomer B) (decomposition point: 148 ~ 150°C) as pale yellow crystals.

7-[2-Cyanomethylene-2-(2-formylaminothiazol-4-yl)acetamido]-3-(1-methyl-1H-tetrazol-5-yl)thiomethyl-3-cephem-4-carboxylic acid (E-isomer):

IR (KBr): 2210, 1775, 1685, 1670 $cm^{-1}$.

NMR δppm ($d_6$-DMSO): 3.73 (bs, 2H), 3.93 (s, 3H), 4.32 (bs, 2H), 5.19 (d, J=5Hz, 1H), 5.81 (dd, J=8Hz, 5Hz, 1H), 6.32 (s, 1H), 7.46 (s, 1H), 8.52 (s, 1H), 9.90 (d, J=8Hz, 1H), 12.5 (bs, 1H).

## Example 6

A methanol solution (1.13 ml) containing 85.7 mg of phosphorus oxychloride was ice-cooled. Subsequently, after 150 mg of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid (E-isomer) was added to the methanol solution, the mixture was stirred for 45 minutes. After 5.6 ml of water was added to the mixture, the resulting mixture was stirred under ice-cooling. The precipitated crystals were collected by filtration, washed with water and dried to obtain 70 mg of 7 - [2 - cyanomethylene - 2 - (2 - aminothiazol - 4 - yl)acetamido] - 3 - (1 - methyl - 1H - tetrazol - 5 - yl)thiomethyl - 3 - cephem - 4 - carboxylic acid hydrochloride salt (decomposition point: 155 ~ 160°C) as pale yellow crystals.

7-[2-Cyanomethylene-2-(2-aminothiazol-4-yl)acetamido]-3-(1-methyl-1H-tetrazol-5-yl)thiomethyl-3-cephem-4-carboxylic acid hydrochloride salt (E-isomer):

IR (KBr): 2220, 1770, 1710, 1665, 1615 $cm^{-1}$.

NMR δppm ($d_6$-DMSO): 3.95 (s, 3H), 4.34 ($q_{AB}$, J=13Hz, 2H), 5.22 (d, J=5Hz, 1H), 5.83 (dd, J=8Hz, 5Hz, 1H), 6.16 (s, 1H), 6.89 (s, 1H), 9.91 (d, J=8Hz, 1H).

## Example 7

To a mixed solution composed of 2.6 ml of trifluoroacetic acid, 1.3 ml of anisole and 4.5 ml of anhydrous methylene chloride, there was added 610 mg of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid benzhydryl ester (Z-isomer) under ice-cooling, and the mixture was stirred for 50 minutes. After 50 ml of ethyl acetate and 20 ml of a 5% aqueous sodium hydroxide solution was added to the mixture, the mixture was separated. The aqueous layer was separated and extracted twice with 10 ml of ethyl acetate. Each ethyl acetate layer was combined, washed twice with water and then washed twice with a saturated saline solution, and dried over magnesium sulfate. After the solution was concentrated, the residue was added small amount of benzene and left to stand. The precipitated crystals were collected by filtration, washed with benzene and dried to obtain 450 mg of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid (Z-isomer) (decomposition point: 128 ~ 132°C) as yellow crystals.

7-[2-Cyanomethylene-2-(2-formylaminothiazol-4-yl)acetamido]-3-(1-methyl-1H-tetrazol-5-yl)thiomethyl-3-cephem-4-carboxylic acid (Z-isomer):

IR (KBr): 2210, 1775, 1710, 1670 $cm^{-1}$.

NMR δppm ($d_6$-DMSO): 3.73 (bs, 2H), 3.94 (s, 3H), 4.33 (bs, 2H), 5.16 (d, J=5Hz, 1H), 5.75 (dd, J=8Hz, 5Hz, 1H), 6.10 (s, 1H), 7.68 (s, 1H), 8.51 (s, 1H), 9.63 (d, J=8Hz, 1H), 12.5 (bs, 1H).

## Example 8

A methanol solution (1.13 ml) containing 85.7 mg of phosphorus oxychloride was ice-cooled. Subsequently, after 150 mg of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid (Z-isomer) was added to the methanol solution, the mixture was stirred for 45 minutes. After 5.6 ml of water and 5 ml of ethyl acetate were added to the mixture, the resulting mixture was adjusted to pH 2.5 with an aqueous sodium hydrogen carbonate solution and separated. The aqueous layer was separated, and washed twice with ethyl acetate. The ethyl acetate layer was combined, washed successive once with water and twice with ethyl acetate, and dried over magnesium sulfate. After the solution was concentrated, the residue was added small amount of benzene and left to stand. The precipitated crystals were collected by filtration, washed with benzene and dried to obtain 105 mg of 7 - [2 - cyanomethylene - 2 - (2 - aminothiazol - 4 - yl)acetamido] - 3 - (1 - methyl - 1H - tetrazol - 5 - yl)thiomethyl - 3 - cephem - 4 - carboxylic acid (Z-isomer) (decomposition point: 115 ~ 120°C) as yellow crystals.

7-[2-Cyanomethylene-2-(2-aminothiazol-4-yl)acetamido]-3-(1-methyl-1H-tetrazol-5-yl)thiomethyl-3-cephem-4-carboxylic acid (Z-isomer):

IR (KBr): 2220, 1775, 1715, 1670, 1610 $cm^{-1}$.

NMR δppm ($d_6$-DMSO): 3.94 (s, 3H), 4.33 ($q_{AB}$, J=13Hz, 2H), 5.17 (d, J=5Hz, 1H), 5.76 (dd, J=8Hz, 5Hz, 1H), 5.98 (s, 1H), 7.12 (s, 1H), 9.78 (d, J=8Hz, 1H).

Example 9

(1) After 700 mg of 7 - [2 - cyanomethylene - 2 - (2 - formylaminothiazol - 4 - yl)acetamido] - 3 - (1 - methyl - 1H - tetrazol - 5 - yl)thiomethyl - 3 - cephem - 4 - carboxylic acid benzhydryl ester was suspended in 3.4 ml of methanol and 7 ml of THF, 2 ml of 35% hydrochloric acid was added to the suspension. Subsequently, the mixture was stirred at room temperature over night. After 5 ml of water was added to the mixture under ice-cooling, the mixture was made to be weak alkaline by adding a saturated aqueous sodium hydrogen carbonate solution, and the resulting mixture was extracted three times with ethyl acetate. The extract was combined, washed successively twice with water and twice with a saturated saline solution, and dried over magnesium sulfate. Then, the solvent in the mixture was removed by evaporation under reduced pressure. The residue was purified by silica gel column chromatography to obtain 370 mg of 7 - [2 - (O - methylcarboxyimidoylmethylene) - 2 - (2 - aminothiazol - 4 - yl)acetamido] - 3 - (1 - methyl - 1H - tetrazol - 5 - yl)thiomethyl - 3 - cephem - 4 - carboxylic acid benzhydryl ester (Z-isomer) as yellow crystals and 170 mg of 7 - [2 - carbamoylmethylene - 2 - (2 - aminothiazol - 4 - yl)acetamido] - 3 - (1 - methyl - 1H - tetrazol - 5 - yl)thiomethyl - 3 - cephem - 4 - carboxylic acid benzhydryl ester (Z-isomer) as pale brown amorphous crystals, respectively.

7-[2-(o-methylcarboxyimidoylmethylene)-2-(2-aminothiazol-4-yl)acetamido]-3-(1-methyl-1H-tetrazol-5-yl)-thiomethyl-3-cephem-4-carboxylic acid benzhydryl ester (Z-isomer):

IR (KBr): 1780, 1715, 1670, 1620 $cm^{-1}$.

NMR δppm ($d_6$-DMSO): 3.68 (s, 3H), 3.78 (bs, 2H), 3.88 (s, 3H), 4.22 (d, J=14Hz, 1H), 4.41 (d, J=14Hz, 1H), 5.23 (d, J=5Hz, 1H), 5.87 (dd, J=8Hz, 5Hz, 1H), 6.38 (s, 1H), 6.83 (s, 1H), 6.92 (s, 1H), 7.08 (bs, 3H), 7.37 (m, 10H), 9.59 (d, J=8Hz, 1H).

7-[2-Carbamoylmethylene-2-(2-aminothiazol-4-yl)acetamido]-3-(1-methyl-1H-tetrazol-5-yl)thiomethyl-3-cephem-4-carboxylic acid benzhydryl ester (Z-isomer):

IR (KBr): 1780, 1720, 1660, 1610 $cm^{-1}$.

NMR δppm ($d_6$-DMSO): 3.74 (bs, 2H), 3.86 (s, 3H), 4.19 (d, J=14Hz, 1H), 4.39 (d, J=14Hz, 1H), 5.17 (d, J=5Hz, 1H), 5.88 (dd, J=8Hz, 5Hz, 1H), 6.59 (s, 1H), 6.77 (s, 1H), 6.88 (s, 1H), 7.34 (m, 12H), 8.06 (bs, 2H), 9.52 (d, J=8Hz, 1H).

(2) A mixed solution composed of 0.43 ml of trifluoroacetic acid, 0.21 ml of anisole and 0.72 ml of methylene chloride was ice-cooled. Subsequently, after 100 mg of 7 - [2 - (O - methylcarboxyimidoylmethylene) - 2 - (2 - aminothiazol - 4 - yl)acetamido] - 3 - (1 - methyl - 1H - tetrazol - 5 - yl)thiomethyl - 3 - cephem - 4 - carboxylic acid benzhydryl ester was added to the mixed solution, the mixture was stirred for 1 hour. Then, 3 ml of ethyl acetate and 1 ml of water were added thereto, and the mixture was adjusted to pH 6.0 ~ 6.5 with a 10% aqueous sodium hydroxide solution under ice-cooling. The aqueous layer was separated, adjusted to pH 2.5 with 1N hydrochloric acid, and extracted with ethyl acetate. The extract was washed successively with water and twice with a saturated saline solution, and dried over magnesium sulfate. Evaporation of the solvent yielded 60 mg of 7 - [2 - (O - methylcarboxyimidoylmethylene) - 2 - (2 - aminothiazol - 4 - yl)acetamido] - 3 - (1 - methyl - 1H - tetrazol - 5 - yl)-thiomethyl - 3 - cephem - 4 - carboxylic acid (decomposition point: 81 ~ 85°C) as pale yellow amorphous crystals.

7-[2-(O-Methylcarboxyimidoylmethylene)-2-(2-aminothiazol-4-yl)acetamido]-3-(1-methyl-1H-tetrazol-5-yl)-thiomethyl-3-cephem-4-carboxylic acid:

IR (KBr): 1780, 1715, 1665, 1630 $cm^{-1}$.

NMR δppm ($d_6$-DMSO): 3.67 (bs, 5H), 3.93 (s, 3H), 4.30 (bs, 2H), 5.12 (d, J=5Hz, 1H), 5.70 (dd, J=8Hz, 5Hz, 1H), 6.43 (s, 1H), 6.84 (s, 1H), 9.54 (d, J=8Hz, 1H).

Example 10

A mixed solution composed of 0.69 ml of trifluoroacetic acid, 0.34 ml of anisole and 1.15 ml of methylene chloride was ice-cooled. Subsequently, after 160 mg of 7 - [2 - carbamoylmethylene - 2 - (2 - aminothiazol - 4 - yl)acetamido] - 3 - (1 - methyl - 1H - tetrazol - 5 - yl)thiomethyl - 3 - cephem - 4 - carboxylic acid benzhydryl ester was added to the mixed solution, the resulting mixture was stirred for 1 hour. Then, 1 ml of water was added thereto, and the resulting mixture was adjusted to pH 6.0 with a 10% aqueous sodium hydroxide solution. After the aqueous layer was washed with ethyl acetate, activated charcoal was added thereto. The mixture was stirred and filtrated through membrane filter. After the filtrate was adjusted to pH 2.3 with 1N hydrochloric acid, the residue was stirred for 1 hour under ice-cooling. The precipitated crystals were collected by filtration, washed with water and dried to obtain 64 mg of 7 - [2 -

carbamoylmethylene - 2 - (2 - aminothiazol - 4 - yl)acetamido] - 3 - (1 - methyl - 1H - tetrazol - 5 - yl)-thiomethyl - 3 - cephem - 4 - carboxylic acid (decomposition point: 106 ~ 110°C).

7-[2-Carbamoylmethylene-2-(2-aminothiazol-4-yl)acetamido]-3-(1-methyl-1H-tetrazol-5-yl)thiomethyl-3-cephem-4-carboxylic acid:

IR (KBr): 1775, 1705, 1670, 1615 $cm^{-1}$.

NMR δppm ($d_6$-DMSO): 3.62 (d, J=18Hz, 1H), 3.80 (d, J=18Hz, 1H), 3.91 (s, 3H), 4.22 (d, J=14Hz, 1H), 4.38 (d, J=14Hz, 1H), 5.14 (d, J=5Hz, 1H), 5.72 (dd, J=8Hz, 5Hz, 1H), 6.46 (s, 1H), 6.98 (s, 1H), 6.2 ~ 7.6 (bs, 1H), 7.98 (bs, 1H), 8.52 (bs, 1H), 8.2 ~ 9.3 (bs, 2H), 9.62 (d, J=8Hz, 1H).

Test Example

Antibacterial activities of the compounds according to this invention, which had been prepared in the preceding Examples, were measured according to the method prescribed by The Committee of Amendment of "The method for measurement of the minimum inhibition concentration", The Chemotherapy Association, Japan (See Chemotherapy, Vol. 29, No. 1, pp. 76 ~ 79 (1981)).

The test results are shown in terms of the minimum inhibition concentration (MIC) (unit: μg/ml) in the following Table 1.

TABLE 1 (MIC)

| Example No. \ Microorganism (strain) | *Staphylococcus aureus* MS 353 | *Salmonella typhimurium* IID—971 | *Proteus morganii* IFO 3848 |
|---|---|---|---|
| 1 | 3.125 | 25 | — |
| 2 | 1.56 | ≦0.006 | ≦0.006 |
| 3 | 0.78 | ≦0.006 | ≦0.006 |
| 4 | 1.56 | ≦0.006 | ≦0.006 |
| 5 | 50 | 25 | 50 |
| 6 | 100 | 0.39 | 0.39 |
| 7 | 1.56 | 0.05 | 0.39 |
| 8 | 0.78 | ≦0.006 | ≦0.006 |
| 9 | 3.125 | 0.0125 | ≦0.006 |
| 10 | 12.5 | 0.10 | ≦0.006 |

**Claims**

1. A compound having the following formula:

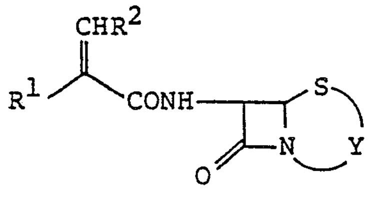

[I]

in which

$R^1$ denotes an aminothiazolyl radical or an aminothiazolylmercapto radical;

$R^2$ denotes a halogen atom, a cyano radical, a $C_1$ to $C_4$-alkylmercapto radical, an O—$C_1$ to $C_4$-alkylcarboxyimidoyl radical or a carbamoyl radical,

provided that the case where $R^1$ denotes an aminothiazolyl radical and $R^2$ denotes a halogen atom is excluded; and

Y represents a group

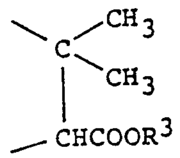

or

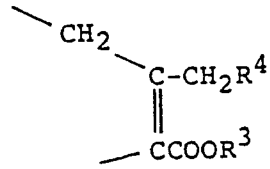

(wherein $R^3$ denotes hydrogen, a salt forming cation or a protective group for a carboxylic acid, and $R^4$ denotes hydrogen or a residue of a nucleophilic compound).

2. A compound according to Claim 1, wherein the compound has the formula:

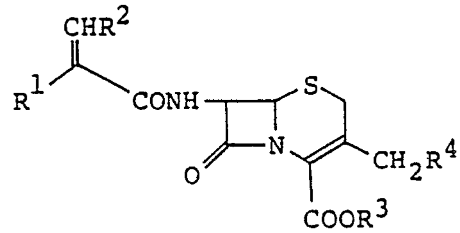

in which $R^1$, $R^2$, $R^3$ and $R^4$ have the same meanings as in Claim 1.

3. A compound according to Claim 1, wherein the compound is selected from the group consisting of sodium 7-[2-chloromethylene-3-(2-aminothiazol-5-yl)mercaptoacetamido]-3-(1-methyl-1H-tetrazol-5-yl)thiomethyl-3-cephem-4-carboxylate,

7-[2-ethylmercaptomethylene-2-(2-aminothiazol-4-yl)acetamido]-3-(1-methyl-1H-tetrazol-5-yl)thiomethyl-3-cephem-4-carboxylic acid (Z-Isomer and E-Isomer),

7-[2-methylmercaptomethylene-2-(2-aminothiazol-4-yl)acetamido]-3-(1-methyl-1H-tetrazol-5-yl)thiomethyl-3-cephem-4-carboxylic acid (Z-Isomer and E-Isomer),

7-[2-cyanomethylene-2-(2-aminothiazol-4-yl)acetamido]-3-(1-methyl-1H-tetrazol-5-yl)thiomethyl-3-cephem-4-carboxylic acid (Z-Isomer),

7-[2-(O-methylcarboxyimidoylmethylene)-2-(2-aminothiazol-4-yl)acetamido]-3-(1-methyl-1H-tetrazol-5-yl)thiomethyl-3-cephem-4-carboxylic acid,

7-[2-carbamoylmethylene-2-(2-aminothiazol-4-yl)acetamido]-3-(1-methyl-1H-tetrazol-5-yl)thiomethyl-3-cephem-4-carboxylic acid.

4. A process for the preparation of a compound having the formula I of claim 1 which comprises reacting a compound having the formula:

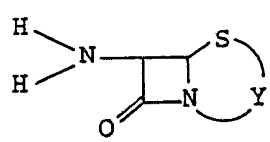

[IV]

wherein Y has the same meaning as defined in claim 1, a reactive derivative thereof with respect to the amino radical or a salt thereof, with an acid having the formula:

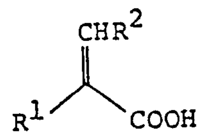

[V]

wherein $R^1$ and $R^2$ have the same meanings as defined in claim 1, a reactive derivative thereof with respect to the carboxyl radical or a salt thereof.

5. A process according to Claim 4, wherein the reaction is carried out in the presence of a dehydration agent or a condensing agent when the acid is reacted in the form of a free acid.

6. A process according to Claim 4, wherein said reactive derivative of the acid is an acid chloride, an acid anhydride, a mixed acid anhydride, an active acid amide, an acid cyanide, an active ester or an acid azide.

7. A process according to Claim 4, wherein the reaction is carried out in the presence of a solvent with cooling or at room temperature.

**Patentansprüche**

1. Verbindung mit der Formel

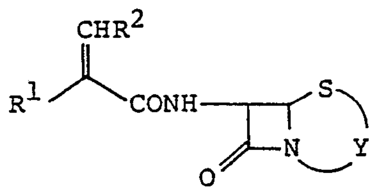

[I]

in der

$R^1$ ein Aminothiazolylradikal oder ein Aminothiazolylmercaptoradikal bedeutet;

$R^2$ bedeutet ein Halogenatom, ein Cyanradikal, ein $C_{1-4}$-Alkylmercaptoradikal, ein O—$C_{1-4}$-Alkylcarboxyimidoylradikal oder ein Carbamoylradikal, vorausgesetzt, das der Fall, bei dem $R^1$ ein Aminothiazolylradikal und $R^2$ ein Halogenatom bedeuten, ausgeschlossen ist; und

Y stellt eine Gruppe

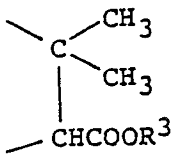

oder

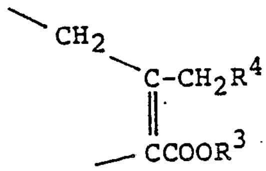

dar (wobei $R^3$ Wasserstoff, ein kationbildendes Salz oder eine Schutzgruppe für eine Carbonsäure bedeutet, und $R^4$ Wasserstoff oder einen Rest einer nukleophilen Verbindung bezeichnet).

2. Verbindung nach Anspruch 1, wobei die Verbindung die Formel

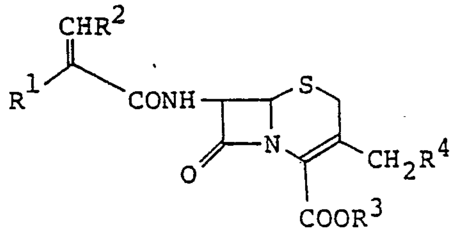

aufweist, in der $R^1$, $R^2$, $R^3$ und $R^4$ die gleichen Bedeutungen wie in Anspruch 1 besitzen.

3. Verbindung nach Anspruch 1, wobei die Verbindung aus der Reihe

Natrium-7-[2-chloromethylen-3-(2-aminothiazol-5-yl)mercaptoacetamido]-3-(1-methyl-1H-tetrazol-5-yl)thiomethyl-3-cephem-4-carboxylat,

7-[2-Ethylmercaptomethylen-2-(2-aminothiazol-4-yl)acetamido]-3-(1-methyl-1H-tetrazol-5-yl)thiomethyl-3-cephem-4-carbonsäure (Z-Isomer und E-Isomer),

7-[2-Methylmercaptomethylen-2-(2-aminothiazol-4-yl)acetamido]-3-(1-methyl-1H-tetrazol-5-yl)thiomethyl-3-cephem-4-carbonsäure (Z-Isomer und E-Isomer),

7-[2-Cyanomethylen-2-(2-aminothiazol-4-yl)acetamido]-3-(1-methyl-1H-tetrazol-5-yl)thiomethyl-3-cephem-4-carbonsäure (Z-Isomer),

7-[2-(O-Methylcarboxyimidoylmethylen)-2-(2-aminothiazol-4-yl)acetamido]-3-(1-methyl-1H-tetrazol-5-yl)thiomethyl-3-cephem-4-carbonsäure,

7-[2-Carbamoylmethylen-2-(2-aminothiazol-4-yl)acetamido]-3-(1-methyl-1H-tetrazol-5-yl)thiomethyl-3-cephem-4-carbonsäure

ausgewählt ist.

4. Verfahren zur Herstellung einer Verbindung der Formel (I) des Anspruches 1, das die Reaktion einer Verbindung mit der Formel

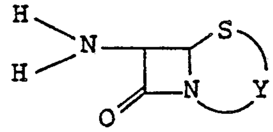

[IV]

wobei Y die Bedeutung wie in Anspruch 1 besitzt, eines reaktiven Derivates davon mit Hinblick auf das Aminoradikal oder eines Salzes davon mit einer Säure mit der Formel

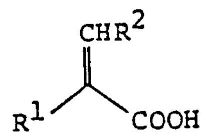

[V]

wobei $R^1$ und $R^2$ die gleichen Bedeutungen wie in Anspruch 1 besitzen, einem reaktiven Derivat davon mit Hinblick auf das Carboxylradikal oder einem Salz davon umfasst.

5. Verfahren nach Anspruch 4, wobei die Reaktion in Gegenwart eines wasserentziehenden Mittels oder eines Kondensiermittels durchgeführt wird, wenn die Säure in Form einer freien Säure umgesetzt wird.

6. Verfahren nach Anspruch 4, wobei das reaktive Derivat der Säure ein Säurechlorid, ein Säureanhydrid, ein gemischtes Säureanhydrid, ein aktives Säureamid, ein Säurecyanid, ein aktiver Ester oder ein Säureazid ist.

7. Verfahren nach Anspruch 4, wobei die Reaktion in Gegenwart eines Lösungsmittels unter Kühlen oder bei Raumtemperatur durchgeführt wird.

**Revendications**

1. Composé répondant à la formule suivante:

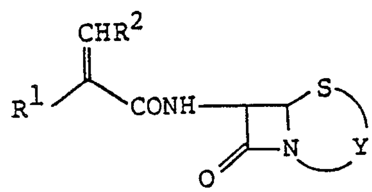

[I]

dans laquelle

$R^1$ désigne un radical aminothiazolyle ou un radical aminothiazolylmercapto;

$R^2$ désigne un atome d'halogène, un radical cyano, un radical (alkyle en $C_1$ à $C_4$)mercapto, un radical O-(alkyle en $C_1$ à $C_4$)carboxyimidoyle ou un radical carbamoyle,

sous réserve que le cas où $R^1$ désigne un radical aminothiazolyle et $R^2$ désigne un atome d'halogène est exclu; et

Y représente un groupe

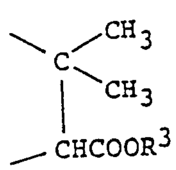

ou

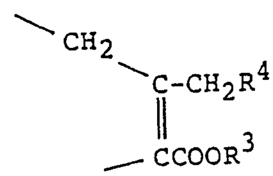

(où $R^3$ désigne un atome d'hydrogène, un cation formant un sel ou un groupe protecteur pour un acide carboxylique et $R^4$ désigne un atome d'hydrogène ou un radical d'un composé nucléophile).

2. Composé selon la revendication 1, répondant à la formule:

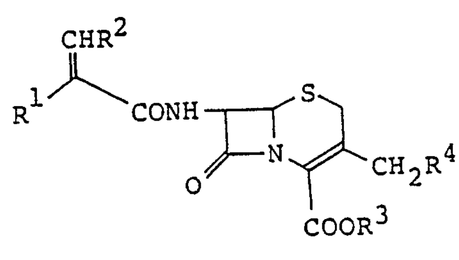

dans laquelle $R^1$, $R^2$, $R^3$ et $R^4$ ont les mêmes significations que dans la revendication 1.

3. Composé selon la revendication 1, qui est choisi dans le groupe constitué

du 7-[2-chlorométhylène-3-(2-aminothiazol-5-yl)-mercaptoacétamido]-3-(1-méthyl-1H-tétrazol-5-yl)-thiométhyl-3-céphème-4-carboxylate de sodium,

de l'acide 7-[2-éthylmercaptométhylène-2-(2-aminothiazol-4-yl)-acétamido]-3-(1-méthyl-1H-tétrazol-5-yl)thiométhyl-3-céphème-4-carboxylique (Isomère Z et Isomère E),

de l'acide 7-[2-méthylmercaptométhylène-2-(2-aminothiazol-4-yl)-acétamido]-3-(1-méthyl-1H-tétrazol-5-yl)thiométhyl-3-céphème-4-carboxylique (Isomère Z et Isomère E),

de l'acide 7-[2-cyanométhylène-2-(2-aminothiazol-4-yl)acétamido]-3-(1-méthyl-1H-tétrazol-5-yl)thiométhyl-3-céphème-4-carboxylique (Isomère Z),

de l'acide 7-[2-(O-méthylcarboxyimidoylméthylène)-2-(2-aminothiazol-4-yl)acétamido]-3-(1-méthyl-1H-tétrazol-5-yl)thiométhyl-3-céphème-4-carboxylique,

de l'acide 7-[2-carbamoylméthylène-2-(2-aminothiazol-4-yl)acétamido]-3-(1-méthyl-1H-tétrazol-5-yl)-thiométhyl-3-céphème-4-carboxylique.

4. Procédé de préparation d'un composé répondant à la formule (I) de la revendication 1 qui comprend la réaction d'un composé répondant à la formule:

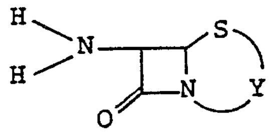

[IV]

dans laquelle Y a la même signification que dans la revendication 1, un de ses dérivés réactifs en ce qui concerne le radical amino ou un de ses sels, avec un acide répondant à la formule:

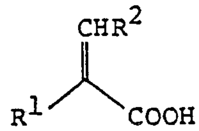

[V]

dans laquelle $R^1$ et $R^2$ ont les mêmes significations que dans la revendication 1, un de ses dérivés en ce qui concerne le radical carboxyle ou un de ses sels.

5. Procédé selon la revendication 4, dans lequel la réaction est effectuée en présence d'un agent de déshydratation ou d'un agent de condensation lorsque l'acide est mis à réagir sous la forme d'un acide libre.

6. Procédé selon la revendication 4, dans lequel ce dérivé réactif de l'acide est un chlorure d'acide, un anhydride d'acide, un anhydride d'acide mixte, un amide d'acide actif, un cyanure d'acide, un ester actif ou un azide d'acide.

7. Procédé selon la revendication 4, dans lequel la réaction est effectuée en présence d'un solvant, en refroidissant ou à la température ambiante.